# EUROPEAN PATENT APPLICATION

(11) **EP 1 769 812 A1**
(43) Date of publication of application: **04.04.2007**
(21) Application number: 06013505.0
(22) Date of filing: 29.06.2006
(51) Int. Cl.: A61M 5/142

(54) **Medical pump with lockout system**

(30) Priority: 30.09.2005 US 241436
(71) Applicant: Sherwood Services AG, 8201 Schaffhausen (CH)
(72) Inventor: Valego, Matthew Edward, North Attleboro, MA 02760 (US); Knauper, Christopher A., O`Fallon, MO 63366 (US); Wiesner, Joel D., St.Peters, MO 63376 (US)
(74) Representative: Tomlinson, Edward James

(57) **Abstract**

A pumping apparatus for administering fluids to a patient. The pump apparatus includes a pump, a controller, and a user interface operatively connected to the controller having a plurality of push buttons for providing inputs to the controller when the push buttons are depressed in an unlock state of the pump. At least some of the inputs are capable of causing the controller to change operation of the pump. At least one (21a) of the plurality of push buttons has multiple functionality and is operable when depressed and released in the unlock state of the pump to provide an input for use in affecting the operation of the controller. The one push button is operable when depressed and held in a depressed position for a predetermined time interval in the unlock state of the pump to initiate a lockout state of the pump.

## Description

### Background of the Invention

This invention relates generally to pumps used to deliver fluids to patients by way of an administration set, and more particularly to a pump having a user interface for initiating a lockout state of the pump.

Administering fluids containing medicine or nutrition to a patient is well known in the art. Typically, fluid is delivered to the patient by a pump set loaded on a flow control apparatus, such as a peristaltic pump, which delivers fluid to the patient at a controlled rate of delivery. A peristaltic pump usually comprises a housing that includes a rotor or the like operatively engaged to at least one motor through a gearbox. The rotor drives fluid through the tubing of the pump set by the peristaltic action effected by rotation of the rotor by the motor. The motor is operatively connected to a rotatable shaft that drives the rotor, which in turn progressively compresses the tubing and drives the fluid at a controlled rate through the pump set. The pump set may have a type of valve mechanism for permitting or preventing fluid flow communication through the pump set. A controller operates the motor or motors used to drive the rotor and, if necessary, control fluid flow as by operation of the valve mechanism. The microprocessor is typically programmable to run for a selected period of time at a selected rate. The pump also may be programmable to permit the operator to select other infusion parameters such as the volume of fluid delivered to the patient. A user interface typically may have a keypad or other device to allow an operator to select the run settings for the pump or program the infusion parameters of a particular run setting.

There is a risk that the pump operating parameters, once programmed into the pump, can be changed as a result of inadvertent contact with the keypad or as a result of purposeful, unauthorized tampering. In particular, when the pump is used in an ambulatory setting, frequent inadvertent contact with the keypad is likely. In either event, the pump may deliver incorrect amounts of fluid or no fluid, which may have deleterious effects on the patient.

### Summary of Invention

In one aspect of the present invention, pumping apparatus for administering fluids to a patient generally comprises a pump for controlling the flow of fluid to the patient and a controller for controlling the operation of the pump. A user interface is operatively connected to the controller comprising a plurality of push buttons for providing inputs to the controller when the push buttons are depressed in an unlock state of the pump. At least some of the inputs are capable of causing the controller to change operation of the pump. At least one of the plurality of push buttons has multiple functionality. The one push button being operable when depressed and released in the unlock state of the pump to provide an input for use in affecting the operation of the controller. The one push button is operable when depressed and held in a depressed position for a predetermined time interval in the unlock state of the pump to initiate a lockout state of the pump in which the push buttons including the one push button are disabled from providing pump operation inputs to the controller.

In another aspect, the present invention is directed to a method of controlling a pumping apparatus comprising a pump for controlling the flow of fluid to the patient, a controller for controlling the operation of the pump, and a user interface operatively connected to the controller. The user interface comprises a plurality of push buttons for providing an input to the controller when the push buttons are depressed in an unlock state of the pump. The method comprises depressing and releasing at least one of the plurality of push buttons in an unlock state of the pump to provide an input to the controller for use in affecting operation of the controller. The one depressed push button is depressed and held in a depressed position in the unlock state of the pump for a predetermined time interval. A lockout state is initiated upon detection of the one push button being held in the depressed position for the time interval in which the push buttons including the one push button are disabled from providing inputs to the controller thereby preventing unintended adjustment to operation of the pump.

Other objects and features of the present invention will be in part apparent and in part pointed out hereinafter.

### Brief Description of the Drawings

Fig. 1 is a perspective of an enteral feeding pump having a display indicating an unlocked state of the pump;

Fig. 2 is a perspective similar to Fig. 1 but showing the display indicating a locked state of the pump;

Fig. 3 is a side elevation thereof showing a fragmentary portion of an administration feeding set received in the pump;

Fig. 4 is a block diagram showing the elements of the pump; and

Fig. 5 is a flow chart of a lockout system of the present invention.

Corresponding reference characters indicate corresponding parts throughout the several views of the drawings.

### Detailed Description of the Preferred Embodiments

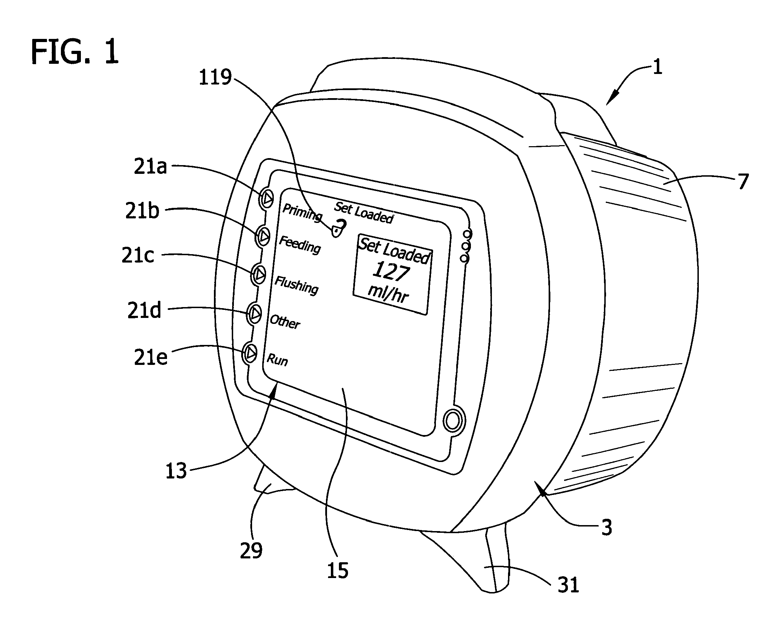

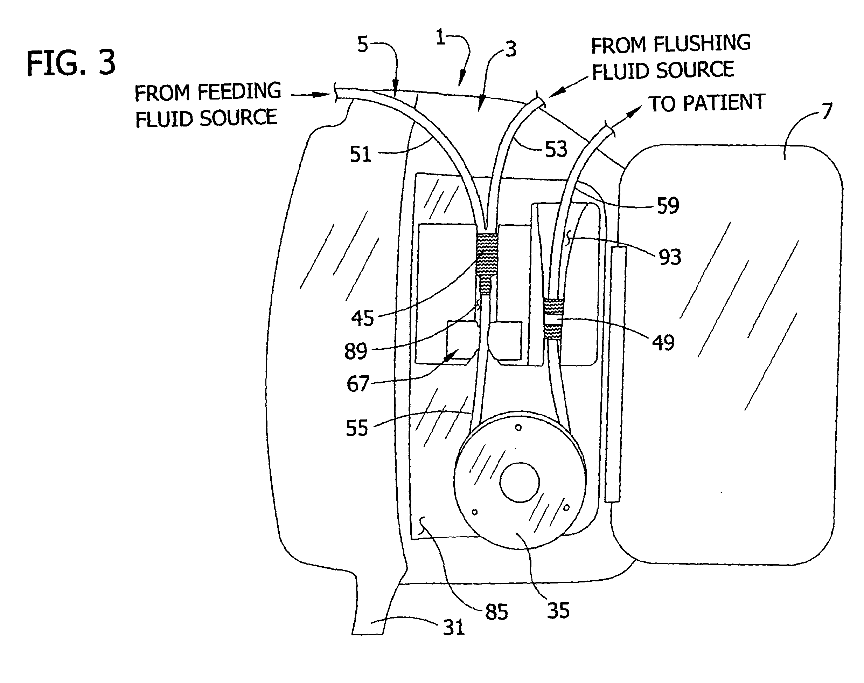

Referring now to the drawings, an enteral feeding pump (broadly, "pumping apparatus") constructed according to the principles of the present invention is generally indicated at 1. The feeding pump 1 comprises a housing, generally indicated at 3, that is constructed so as to mount an administration feeding set (broadly, "a pump set") generally indicated at 5. A fragmentary portion of the feeding set is shown in Fig. 3. Suitable pump sets are shown in co-assigned U.S. Patent Application No. 10/853,958 filed May 24, 2004 entitled FLOW CONTROL APPARATUS, the disclosure of which is incorporated by reference herein. The housing 3 includes a door 7 hinged to the remainder of the housing for swinging between a closed position (Fig. 1) and an open position (Fig. 3) which exposes a portion of the pump 1 that receives the administration feeding set 5. The pump 1 has a user interface, generally indicated at 13, including a display screen 15 on the front of the housing 3 that is capable of displaying information about the status and operation of the pump and a plurality of push buttons 21 a, 21 b, 21 c, 21d, and 21e, on the side of the display screen. The push buttons 21 a thru 21e are provided for use in controlling and obtaining information from the pump 1. In the illustrated embodiment, the push buttons 21 a thru 21e are used for selecting a respective operating mode of the pump such as the "PRIMING", "FEEDING", "FLUSHING", and "RUN" modes of the pump. Legs 29, 31 at the bottom front of the housing 3 support the housing so that the display screen 15 is angled slightly upward for ease of viewing.

It will be understood that although the illustrated pump 1 is an enteral feeding pump, the present invention has application to other types of pumping apparatus, including medical infusion pumps. The pump 1 has a rotor 35 (Fig. 3) in the housing 3 that controls the flow of fluid through the feeding set 5. The general construction an operation of the enteral feeding pump 1, except as set forth hereinafter, may be generally the same as disclosed in co-assigned U.S. Patent Application Nos. 10/854,136 filed May 24, 2004 and entitled FLOW CONTROL APPARATUS, and 10/853,926 filed May 25, 2004 entitled FLOW MONITORING SYSTEM FOR A FLOW CONTROL APPARATUS, the disclosures of which are incorporated herein by reference. Moreover, although an administration feeding set 5 is shown, other types of pump sets (not shown) can be used within the scope of the present invention.

As shown in Fig. 3, the administration feeding set 5 includes a valve mechanism 45 and a mounting collar 49 that are loaded in the pump 1 for delivery of fluid to a patient. The feeding set includes a first section of tubing 51 upstream of the valve mechanism 45 leading to a feeding fluid source (not shown) and a second section of tubing 53 upstream of the valve mechanism leading to a flushing fluid source (not shown). The feeding set 5 includes a third section of tubing 55 downstream of the valve mechanism connecting the valve to the mounting collar 49 and a fourth portion of tubing 59 leading from the mounting collar 49 to the patient. The valve mechanism 45 is operable to selectively permit flow of feeding fluid from the feeding fluid source (not shown) or a flushing fluid source, or prevent any fluid flow communication from the feeding or flushing fluid sources into the tubing 59 leading to the patient. When loaded into the pump 1, the valve mechanism 45 and the mounting collar 49 are securely engaged with the pump and the third section of tubing 55 is placed in a stretched condition between the valve mechanism 45 and the mounting collar 49 around the rotor 35 of the pump. The valve mechanism 45 may be similar to the valve mechanism is disclosed in co-assigned U.S. Patent Application Serial No. 10/853,958 previously incorporated herein by reference. Various other operational features of the pump are disclosed in co-assigned U.S. Patent Application Serial No. 10/854,008 filed May 25, 2004 entitled RE-CERTIFICATION SYSTEM FOR A FLOW CONTROL APPARATUS, the disclosure of which is incorporated herein by reference.

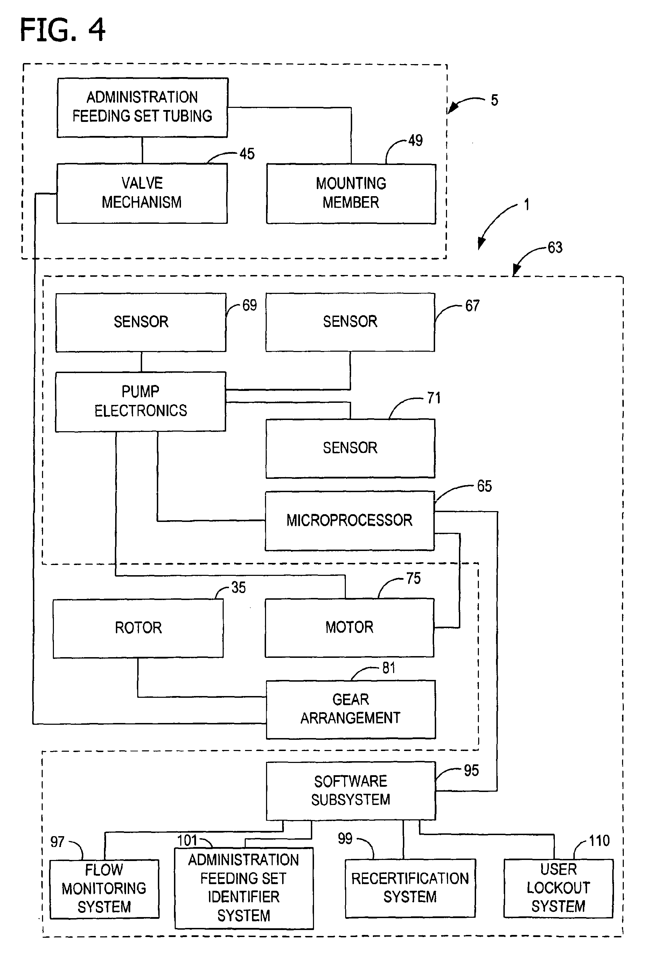

The pump 1 can be programmed or otherwise controlled for operation in a desired manner. For instance, the pump 1 can begin operation to provide feeding fluids to the patient. The caregiver may select (for example) the amount of fluid to be delivered, the rate at which the fluid is to be delivered and the frequency of fluid delivery. The pump 1 has a controller, generally indicated at 63 (Fig. 4), including a microprocessor 65 (Fig. 4) that allows it to accept programming and/or to include preprogrammed operational routines that can be initiated by the caregiver. The microprocessor 65 is in communication with pump electronics that include a flow monitoring sensor, generally indicated at 67, that detects if fluid is flowing at the prescribed setting of the pump. Other sensors, such as an administration set positioning sensor 69 (Fig. 4) that detects whether the administration feeding set 5 has been positioned properly or a sensor 71 that determines the type of administration set that has been placed in the pump 1 can be in communication with the microprocessor to facilitate accurate operation of the pump. The controller 63 also controls the operation of a pump motor 75 that operates the rotor and the valve mechanism 45 through a gear unit 81. The pump motor 75 can operate the valve mechanism 45 and rotor 35 independently of each other.

The housing 3 of the pump 1 has an interior space 85 adapted for receiving the administration feeding set 5. The interior space 85 of the housing 3 is selectively enclosed by the door 7 mounted on the housing. The interior space 85 of the pump has a first chute 89 and a second chute 93 for receiving respective portions of the administration feeding set 5. The first chute 89 receives the valve mechanism 45 and the second chute 93 receives the mounting collar 49. The rotor 35 is located in the interior space 85 below the first and second chutes 89, 93 and engages the third section of the tubing 55 when the tubing section is placed in a stretched condition between the first and second chutes. Rotation of the rotor 35 compresses tubing 55 and provides a force for driving fluid in the feeding set 5 from the upstream side of the rotor to the downstream side of the rotor for delivery to the patient. The sensor 67 in the housing 5 is located in a position to detect the presence or absence of fluid in the upstream side of the tubing 55 of the feeding set 5.

The microprocessor 65 includes a software subsystem 95 in memory of the controller 63 that includes a flow monitoring system 97 that determines through a series of decision points and steps whether normal or abnormal flow conditions exist within the administration feeding set 5, and if an abnormal flow condition does exist, whether it is a bag empty condition, upstream occlusion, or downstream occlusion. The software subsystem 95 may include other systems such as a recertification system 99, administration feeding set identifier system 101, or other systems to control various aspects of the pump 1.

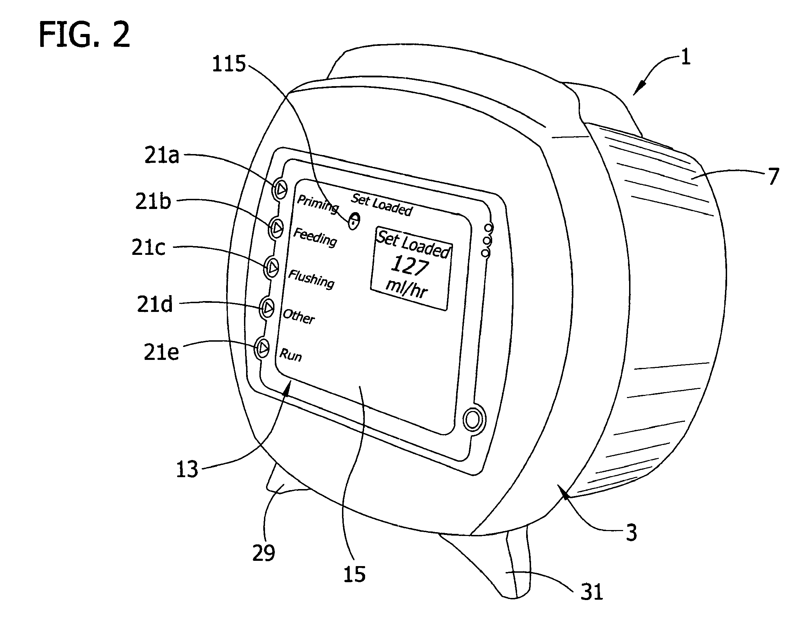

The software subsystem 95 has a lockout system 110 that is initiated by a caregiver or user of the pump 1. The lockout system 110 places the pump 1 in a locked state in which the push buttons 21a thru 21 e on the user interface 13 are deactivated to prevent the operation of the pump from being accidentally or inadvertently changed. Further, the lockout system 110 prevents the pump 1 from being turned off when the pump is in the locked state. As shown in Fig. 2, the display screen 15 displays a symbol in the form of a locked padlock 115 when the lockout system 110 is initiated and the pump is in the locked state. As shown in Fig. 1, the display screen 15 displays a symbol in the form of an unlocked padlock 119 when the lockout system is not initiated and the pump 1 is in an unlocked state. It is understood that the symbols 115, 119 indicating the locked and unlocked states of the pump 1 may be other than a padlock (e.g., a key, a letter "L", or any other icon that indicates a locked condition) without departing from the scope of this invention.

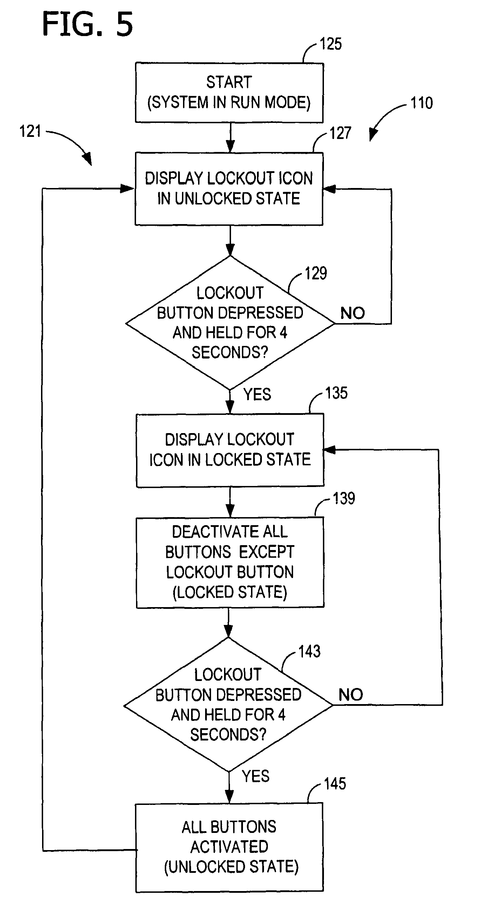

As shown in Fig. 5, the lockout system 110 includes computer-executable instructions 121 that include various decision points and steps that determine whether the pump 1 is in the locked state or the unlocked state. Process block 125 of the instructions 121 determines when the pump 1 is activated and running under normal operating conditions in the "RUN" mode. As indicated by process block 127, the display screen 15 will display the unlocked state indicator 119 when the pump is started in the "RUN" mode. The instructions 121 move to decision block 129 which determines if the lockout button 21a is depressed and held for four seconds. In the illustrated embodiment, the lockout push button 21a is located in the upper left-hand corner of the user interface 13 and is a multi-functional push button that is used to select the "PRIMING" mode of the pump and is used to initiate the lockout system 110 of the microprocessor 65. This push button 21a is referred to as the lockout push button in that it is used by the operator to initiate the lockout system to transfer the pump 1 from the unlocked state to the locked state. When the push button 21a is depressed for a predetermined period of time (e.g. between three and five, preferably four seconds) the instructions 121 proceed to process block 135 that displays the lockout icon 115 in the locked state and then process block 139 that deactivates all push buttons on the user interface except for the lockout push button 21 a. In the locked state, the display screen 15 displays the locked state indicator 115 (Fig. 2) and the microprocessor 65 continues to run the pump in the normal mode with the previously established settings. If the lockout push button 21a has not been depressed for four seconds, the instructions 121 proceed back to the process block 127 which maintains the lockout icon 119 in the unlocked state.

When the locked state has been entered at process block 139, the instructions 121 proceed to decision block 143 that determines if the lockout key 2 1 a has been depressed for four seconds. When the lockout push button 21a is depressed for four seconds, the instructions proceed to process block 145 which activates all the push buttons on the pump 1. After the push buttons are activated, process block 127 of the instructions 121 displays the unlocked state indicator 119 on the display screen 15. From this point the instructions 121 repeat the above sequence until the pump 1 is removed from the "RUN" mode or is powered off.

It is understood that the predetermined period of time at decision block 129 to depress the lockout push button 21a for activation of the lockout system 110 is at least about two seconds, and in one preferred embodiment is about four seconds. The length of time of the predetermined time period should be long enough to prevent inadvertent initiating of the lockout system 110 during normal operation of the lockout key 21a to select the "PRIMING" mode. It is understood that the predetermined time period may be more or less than two seconds without departing from the scope of this invention and that the predetermined time period in decision block 143 required for switching the pump 1 to the unlocked state may be more or less than the predetermined time period in decision block 129 for switching the pump to the locked state.

The lockout push button 21a has multiple functionality in that it is operable when depressed and released in the unlock state of the pump to provide an input to the microprocessor 65 that changes the operating mode of the pump 1 to the "PRIMING" mode. Accordingly, the lockout push button 21 a is normally pressed and released to provide an input to the microprocessor for use in affecting operations of the pump. The lockout push button 21 a is also operable when depressed and held in the depressed position for the predetermined time interval in the unlock state of the pump 1 to initiate the lockout system 110 of the software subsystem 95. Further, the lockout push button 21 a is operable when depressed and held in the depressed position for the predetermined time interval in the lock state of the pump 1 to activate the other push buttons and change the status of the pump to the unlock state. It is understood that the predetermined time intervals for depressing the lockout push button 21 a to initiate the lock state could be more or less than the predetermined time interval required to re-initiate the unlock state of the pump.

In the illustrated embodiment, the lockout push button 21a of the present invention is a single, multi-functional push button on the user interface of the pump 1. It is noted that the use of a single push button as the lockout push button allows the lockout system 110 of the pump to be easily and quickly initiated by the caregiver or user of the pump 1. It is contemplated that the lockout system could require more than one lockout push button to be depressed and held for a predetermined period of time without departing from the scope of this invention.

A method of controlling the enteral feeding pump 1 includes depressing and releasing the lockout push button 21a in the unlock state of the pump to provide an input to the controller 63 for use in affecting operation of the controller. In the illustrated embodiment, the lockout push button 21a is used to select the "PRIMING" function of the pump 1 so that the pump is primed when the lockout push button is depressed and released. Depressing and holding the lockout push button 21a in a depressed position in the unlock state of the pump 1 for a predetermined time interval initiates activation of the lockout system 110 of the microprocessor 65. The lockout system 110 puts the pump in the lockout state upon detection of the lockout push button being held in the depressed position for the predetermined time interval. As noted above, in the lockout state of the pump 1 the operator controls on the user interface 13, including push buttons 21a thru 21e, are disabled from providing inputs to the controller thereby preventing unintended adjustment to operation of the pump. Also, in the lockout state the lockout icon 115 is displayed on the screen to notify the user that the push buttons 21a thru 21 e are disabled and that the pump must be placed in the unlock state in order to make any adjustments to the operation of the pump 1.

To re-initiate the unlock state of the pump 1, the lockout push button 21a is depressed and held for the predetermined time interval in the lockout state of the pump. The lockout push button sends a signal to the microprocessor 65 to enable the push buttons 21a thru 21e on the user interface 13. When the push buttons are enabled, an authorized user or caregiver may control the operation of the pump by using the push buttons 21a thru 21e that provide inputs to the controller to change the operating mode of the pump or change the operating parameters of the pump. When the unlock state of the pump 1 is re-initiated, the unlock icon 119 is displayed on the screen 13.

It is understood that the lock state icon 115 and unlock icon 119 may flash or be displayed intermittently for a uniform time period (e.g., about 1 second) during the operation of the pump 1. Also, the lock state icon 115 and unlock state icon 119 may be positioned on the same portion of the display 15 as other icons, alarm messages, or other status messages that share the same location on the display. In one embodiment, the respective icon 115, 119 may alternate with a second icon or message such as a "SET IN USE > 24 HOURS" message that is displayed on the screen when the pump set has been in use for greater than 24 hours. In this embodiment, the icon 115, 119 displaying the status of the lockout system will alternate with the "SET IN USE > 24 HOURS" message if the controller 63 indicates that the set has been in use greater than 24 hours. It is understood that the icon 115, 119 may intermittently be displayed with other messages or display icons on the same location of the display 15 without departing from the scope of this invention.

The controller 63 typically has at least some form of computer readable media. Computer readable media, which include both volatile and nonvolatile media, removable and non-removable media, may be any available medium that may be accessed by controller 63. By way of example and not limitation, computer readable media comprise computer storage media and communication media. Computer storage media include volatile and nonvolatile, removable and non-removable media implemented in any method or technology for storage of information such as computer readable instructions, data structures, program modules or other data. For example, computer storage media include RAM, ROM, EEPROM, PROM, flash memory or other memory technology, CD-ROM, digital versatile disks (DVD) or other optical disk storage, magnetic cassettes, magnetic tape, magnetic disk storage or other magnetic storage devices, or any other medium that may be used to store the desired information and that may be accessed by controller 63. Communication media typically embody computer readable instructions, data structures, program modules, or other data in a modulated data signal such as a carrier wave or other transport mechanism and include any information delivery media. Those skilled in the art are familiar with the modulated data signal, which has one or more of its characteristics set or changed in such a manner as to encode information in the signal. Wired media, such as a wired network or direct-wired connection, and wireless media, such as acoustic, RF, infrared, and other wireless media, are examples of communication media. Combinations of any of the above are also included within the scope of computer readable media.

Embodiments of the invention may be described in the general context of computer-executable instructions, such as program modules, executed by one or more computers or other devices. The computer-executable instructions may be organized into one or more computer-executable components or modules including, but not limited to, routines, programs, objects, components, and data structures that perform particular tasks or implement particular abstract data types. Aspects of the invention may be implemented with any number and organization of such components or modules. For example, aspects of the invention are not limited to the specific computer-executable instructions or the specific components or modules illustrated in the figures and described herein. Other embodiments of the invention may include different computer-executable instructions or components having more or less functionality than illustrated and described herein.

Further, the order of execution or performance of the operations in embodiments of the invention illustrated and described herein is not essential, unless otherwise specified. That is, the operations may be performed in any order, unless otherwise specified, and embodiments of the invention may include additional or fewer operations than those disclosed herein. For example, it is contemplated that executing or performing a particular operation before, contemporaneously with, or after another operation is within the scope of aspects of the invention.

In operation, microprocessor 65 of the controller 63 executes computer-executable instructions such as those illustrated in the figures to implement aspects of the invention. Aspects of the invention may also be practiced in distributed computing environments where tasks are performed by remote processing devices that are linked through a communications network. In a distributed computing environment, program modules may be located in both local and remote computer storage media including memory storage devices.

When introducing elements of the present invention or the preferred embodiment(s) thereof, the articles "a", "an", "the" and "said" are intended to mean that there are one or more of the elements. The terms "comprising", "including" and "having" are intended to be inclusive and mean that there may be additional elements other than the listed elements.

As various changes could be made in the above constructions without departing from the scope of the invention, it is intended that all matter contained in the above description or shown in the accompanying drawings shall be interpreted as illustrative and not in a limiting sense. Further, all dimensional information set forth herein is exemplary only and is not intended to limit the scope of the invention. It is understood that any of the particular embodiments of the present invention may include one or more of the aspects or features of the invention as described herein and illustrated in the drawings.

## Claims

1. A pumping apparatus for administering fluids to a patient, the pumping apparatus comprising:
a pump (1) for controlling the flow of fluid to the patient;
a controller (63) for controlling the operation of the pump;
a user interface (13) operatively connected to the controller comprising a plurality of push buttons (21a-21e) for providing inputs to the controller when said push buttons are depressed in an unlock state of the pump, at least some of the inputs being capable of causing the controller to change operation of the pump;
at least one of said plurality of push buttons having multiple functionality, said one push button (21a) being operable when depressed and released in the unlock state of the pump to provide an input for use in affecting the operation of the controller, said one push button being operable when depressed and held in a depressed position for a predetermined time interval in the unlock state of the pump to initiate a lockout state of the pump in which the push buttons including said one push button are disabled from providing inputs to the controller.

2. The pumping apparatus as set forth in claim 1 wherein said time interval is at least 2 seconds.

3. The pumping apparatus as set forth in claims 1 or 2 wherein said time interval is between 3 and 5 seconds.

4. The pumping apparatus as set forth in any of the preceding claims wherein said user interface (13) further comprises a display (15) for displaying a status of the pump (1), said display including a lockout indicator (119) that indicates when the push buttons are disabled.

5. The pumping apparatus as set forth in claim 4 wherein said lockout indicator (119) is located generally near an upper corner of the display (15).

6. The pumping apparatus as set forth in claims 4 or 5 wherein said lockout indicator (119) is a symbol in the shape of a lock.

7. The pumping apparatus set forth in any one of claims 4-6 wherein said lockout indicator (119) displayed intermittently during operation of the pump (1).

8. The pumping apparatus set forth in any one of claims 4-7 wherein said lockout indicator (119) is replaced with a second indicator selected from a group consisting of an icon, an alarm message, or a status message when the lockout indicator is not displayed.

9. The pumping apparatus as set forth in any one of claims 4-8 wherein said at least one push button (21a) is located generally adjacent said lockout indicator (119).

10. The pumping apparatus as set forth in any one of the preceding claims wherein said at least one push button (2 1 a) provides an input for selecting an operating mode of the pump (1) when the push button is depressed and released in the unlock state of the pump.

11. The pumping apparatus as set forth in any one of the preceding claims wherein said at least one push button (21a) is operable when depressed and held in a depressed position for a predetermined time interval in the lockout state of the pump (1) to re-initiate the unlock state of the pump in which the push buttons (21a-21e) are enabled for providing inputs to the controller (63).

12. A method of controlling a pumping apparatus comprising a pump (1) for controlling the flow of fluid to the patient, a controller (63) for controlling the operation of the pump, and a user interface (13) operatively connected to the controller comprising a plurality of push buttons (21a-21e) for providing an input to the controller when said push buttons are depressed in an unlock state of the pump, the method comprising:
depressing and releasing at least one (21a) of said plurality of push buttons (21a-21 e) in an unlock state of the pump to provide an input to the controller for use in affecting operation of the controller;
depressing and holding said one depressed push button in a depressed position in the unlock state of the pump for a predetermined time interval;
initiating a lockout state upon detection of said one push button being held in the depressed position for said time interval in which the push buttons including said one push button are disabled from providing inputs to the controller thereby preventing unintended adjustment to operation of the pump.

13. The method as set forth in claim 12 wherein depressing and releasing said one push button (21a) in the unlock state of the pump (1) includes providing an input to the controller (63) for use in affecting operation of the pump.

14. The method as set forth in claims 12 or 13 further comprising depressing and holding at least one of said plurality of push buttons (21a) in the lockout state of the pump (1), and re-initiating the unlock state upon detection of said one push button being held in the depressed position for said time interval in the lockout state in which the push buttons are enabled for providing inputs to the controller (63).

15. The method as set forth in any one of claims 12-14 wherein holding said one push button (21a) in the depressed position comprises holding the push button in the depressed position for at least 2 seconds.

16. The method as set forth in claim 15 wherein holding said one push button (21a) in the depressed position comprises holding the push button in the depressed position for between 3 and 5 seconds.
